# EUROPÄISCHE PATENTANMELDUNG

(11) **EP 1 562 094 A2**
(43) Veröffentlichungstag der Anmeldung: **10.08.2005**
(21) Anmeldenummer: 05001529.6
(22) Anmeldetag: 26.01.2005
(51) Int. Cl.: G05D 7/06

(54) **Mikrodosiervorrichtung**

(30) Priorität: 05.02.2004 DE 102004006451
(71) Anmelder: Ing. Erich Pfeiffer GmbH, 78315 Radolfzell (DE)
(72) Erfinder: Körner, Joachim, 88690 Uhldingen (DE); Helmlinger, Michael, 78315 Radolfzell (DE); Schürle, Holger, 78315 Radolfzell (DE)
(74) Vertreter: Patentanwälte Ruff, Wilhelm, Beier, Dauster & Partner

(57) **Zusammenfassung**

Eine Mikrodosiervorrichtung (1) für ein Medium mit einem Energiespeicher (2) für elektrische Energie, einem Mediumspeicher (3) und einem Dosierraum (4) zur zumindest zeitweisen Aufnahme einer Flüssigkeitsmenge, dem wenigstens eine Austragöffnung (5) und eine Vibrationseinheit (6) zugeordnet ist, die wenigstens mit einer Begrenzungsfläche (7) des Dosierraums (4) in Wirkverbindung steht, um diese für einen Austragvorgang in Schwingungen zu versetzen, wobei sich eine im Energiespeicher gespeicherte Energiemenge und ein zum Austrag einer im Mediumspeicher bevorrateten Mediummenge benötigter Energiebedarf zumindest nahezu entsprechen.

## Beschreibung

Die Erfindung betrifft eine Mikrodosiervorrichtung für ein Medium mit einem Energiespeicher für elektrische Energie, einem Mediumspeicher und einem Dosierraum zur zumindest zeitweisen Aufnahme einer Flüssigkeitsmenge, dem wenigstens eine Austragöffnung und eine Vibrationseinheit zugeordnet ist, die wenigstens mit einer Begrenzungsfläche des Dosierraums in Wirkverbindung steht, um diese für einen Austragvorgang in Schwingungen zu versetzen.

Derartige Mikrodosiervorrichtungen sind aus dem Stand der Technik in vielfältigen Ausführungsformen bekannt. Sie werden für den Austrag von Flüssigkeiten verwendet und dienen zur Verabreichung von kosmetischen oder pharmazeutischen Produkten an Benutzer. Gattungsgemäße Mikrodosiervorrichtungen weisen einen Mediumspeicher zur Aufnahme der auszutragenden Flüssigkeit und einen mit dem Mediumspeicher verbundenen Dosierraum auf. Der Dosierraum kann durch eine Vibrationseinheit in Schwingungen versetzt werden und beaufschlagt damit eine in dem Dosierraum eingebrachte Flüssigkeit mit Druckwellen. Dazu wird der Vibrationseinheit elektrische Energie zugeführt, die aus einem Energiespeicher der Mikrodosiervorrichtung entnommen wird. Durch die Druckwellen kann die Flüssigkeit unter Überwindung eines Strömungswiderstandes durch zumindest eine Austragöffnung des Dosierraums in eine Umgebung der Mikrodosiervorrichtung abgegeben werden.

Die Aufgabe der Erfindung besteht darin, eine Mikrodosiervorrichtung der eingangs genannten Art zu schaffen, die mit einfachen Mitteln eine wirtschaftliche Funktion zulässt.

Diese Aufgabe wird dadurch gelöst, dass sich eine im Energiespeicher gespeicherte Energiemenge und ein zum Austrag einer im Mediumspeicher bevorrateten Mediummenge benötigter Energiebedarf zumindest nahezu entsprechen. Ein Energiespeicher kann insbesondere als Batterie, Kondensator, Solarzelle, Brennstoffzelle oder Akkumulator oder als Kombination davon ausgeführt sein. Im Energiespeicher ist eine für einen Austragvorgang abrufbare Energiemenge, insbesondere für eine Versorgung der Vibrationseinheit und einer eventuell vorgeschalteten Steuereinheit gespeichert. Diese Energiemenge entspricht zumindest nahezu einem Energiebedarf zur Förderung und zum Austrag des im Mediumspeicher bevorrateten Mediums. Dabei wird der Energiebedarf insbesondere durch die Vibrationseinheit und durch die Steuereinheit bestimmt. Durch die erfindungsgemäße Lösung ist es gewährleistet, dass die Mediummenge in dem Mediumspeicher und die Energie des Energiespeichers gemeinsam zur Neige gehen und demzufolge gemeinsam ausgetauscht bzw. in einem gemeinsamen Arbeitsgang von der Mikrodosiervorrichtung entfernt werden können. Durch eine Entsprechung der gespeicherten Energiemenge mit dem Energiebedarf kann eine besonders günstige Anpassung des Energiespeichers auf die Mediummenge oder in umgekehrter Weise der Mediummenge auf den Energiespeicher vorgenommen werden. Damit kann eine Unter- oder Überdimensionierung des Energiespeichers bzw. des Mediumspeichers vermieden werden. Bei der Anpassung von Energiemenge und Energiebedarf ist zu berücksichtigen, dass durch Entladung oder Alterung des Energiespeichers oder andere äußere Umstände ein Energieverlust auftreten könnte und demzufolge eine gewisse Überkapazität der im Energiespeicher gespeicherten Energiemenge gegenüber dem Energiebedarf vorliegen sollte. Energiemenge und Energiebedarf müssen sich daher nicht vollständig entsprechen. Dadurch kann ein sicherer Austrag der gesamten Mediummenge aus dem Mediumspeicher gewährleistet werden.

In Ausgestaltung der Erfindung sind der Energiespeicher und der Mediumspeicher in einer diskreten Verbrauchseinheit zusammengefasst. Damit kann gewährleistet werden, dass der auf die Mediummenge abgestimmte Energiespeicher auch tatsächlich im wesentlichen proportional mit dem Mediumaustrag entleert wird und somit die gewünschte Beziehung zwischen gespeicherter Energiemenge und benötigtem Energiebedarf erhalten bleibt. Für eine Zusammenfassung des Energiespeichers und des Mediumspeichers in der Verbrauchseinheit kann eine übergeordnete Baueinheit im Sinne eines gemeinsamen Gehäuses vorgesehen werden. In dieses gemeinsame Gehäuse werden dann übliche Mediumspeicher und Energiespeicher eingebaut und somit zu der Verbrauchseinheit zusammengefügt. In einer Ausführungsform sind der Energiespeicher und der Mediumspeicher als integrale Bestandteile der Verbrauchseinheit unlösbar miteinander verbunden. Bei einer weiteren, besonders bevorzugten Ausführungsform ist die Verbrauchseinheit lösbar mit einem Gehäuse der Mikrodosiervorrichtung verbunden. Vorteilhaft ist für eine An- und Abkopplung der Verbrauchseinheit eine elektromechanische Schnittstelle mit Verriegelungsmitteln und Kontaktiermitteln vorgesehen. Als Verriegelungsmittel können dabei insbesondere form-, stoff- und/oder kraftschlüssig wirkende Riegeleinrichtungen wie Rastnasen, Hinterschnitte, Führungsschienen oder Magnetelemente vorgesehen sein. Als Kontaktiermittel zur Übertragung elektrischer Signale können insbesondere Kontaktierflächen und korrespondierende Kontaktzungen oder Kontaktfedern vorgesehen werden. Die elektromechanische Schnittstelle ermöglicht eine feste, an- und abkoppelbare Verbindung der Verbrauchseinheit zu der Mikrodosiervorrichtung. Dabei kann zeitgleich mit einer mechanischen Anbringung der Verbrauchseinheit an die Mikrodosiervorrichtung auch eine elektrische Kontaktierung zwischen Mikrodosiervorrichtung und Verbrauchseinheit hergestellt werden.

In weiterer Ausgestaltung der Erfindung ist an dem Mediumspeicher eine manuell zu betätigende Dosierpumpe vorgesehen. Eine manuell zu betätigende Dosierpumpe kann insbesondere als Kolbenpumpe, Membranpumpe oder Tropfenspenderpumpe ausgeführt sein und ist vorzugsweise als Pumpsystem für ein konservierungsmittelfreies Medium verwirklicht. Die Dosierpumpe dient zum Transport des im Mediumspeicher bevorrateten Mediums zum Dosierraum. Durch die Verwendung einer manuell zu betätigenden Dosierpumpe kann eine großindustriell herstellbare und damit besonders kostengünstige Dosierpumpe eingesetzt werden, die nur geringe Anpassungen auf die Mikrodosiervorrichtung erfordert. Zur Betätigung der Dosierpumpe ist an der Mikrodosiervorrichtung eine Handhabe, insbesondere als Schieber oder Drucktaste ausgeführt, vorgesehen. Mit dieser Handhabe kann der Benutzer eine Kraft auf die Dosierpumpe ausüben, wodurch eine definierte Mediummenge in den Dosierraum eingebracht wird. Die Ausbringung des Mediums kann abhängig oder unabhängig von der aufgebrachten Benutzerkraft erfolgen.

In weiterer Ausgestaltung der Erfindung ist eine mechanische und/oder elektrische Dosiertriggerung zur Synchronisation der Dosierung mit einer Atembewegung eines Benutzers vorgesehen. Bei einer Verwendung der Mikrodosiervorrichtung zur Abgabe von insbesondere pharmazeutischen oder medizinischen Inhaltsstoffen, insbesondere in einen Atemtrakt eines Benutzers durch Inhalation, ist eine Dosiertriggerung vorteilhaft. Mittels der Dosiertriggerung kann eine von der Mikrodosiervorrichtung abzugebende Mediummenge genau dann abgegeben werden, wenn eine Atembewegung eines Benutzers durch die Dosiertriggerung festgestellt wird. Damit kann die ordnungsgemäße Inhalation des von der Mikrodosiervorrichtung ausgetragenen Mediums durch den Benutzer gewährleistet werden. Eine mechanische und/oder elektrische Dosiertriggerung kann insbesondere durch eine volumenstromabhängige Auslösung der Dosierung verwirklicht werden. Als Volumenstrom wird eine Luftmenge pro Zeiteinheit ermittelt, die insbesondere vom Benutzer durch Inhalation durch die Mikrodosiervorrichtung hindurch angesaugt wird. Zur Ermittlung des Volumenstroms können an der Mikrodosiervorrichtung entweder mechanische Klappenventile durch eine bei der Atembewegung auftretende Ansaugströmung ausgelöst werden. Alternativ oder ergänzend kann eine elektrische und/oder elektronische Volumenstrommessung, insbesondere durch kapazitive, resistive oder induktive Volumenstrommessung vorgesehen werden. Sobald ein Grenzwert für den Luftstrom erreicht ist, löst die Dosiertriggerung auf mechanischem, elektromechanischem oder elektronischem Wege die Dosierung des Mediums aus. Dadurch wird die gewünschte Synchronisierung zwischen der Atembewegung des Benutzers und der Dosierung erreicht.

In weiterer Ausgestaltung der Erfindung ist eine mechanische und/oder elektronische Dosierverriegelung zur Sicherstellung einer korrekten Mediumdosierung vorgesehen. Durch eine Dosierverriegelung kann eine Anwendung der Mikrodosiervorrichtung durch unbefugte Benutzer verhindert werden. Weiterhin kann durch Integration eines Zeitglieds in die Dosierverriegelung eine zeitliche Steuerung von aufeinander folgenden Dosiervorgängen ermöglicht werden. Eine Dosierverriegelung kann auch im Falle einer Überalterung des auszutragenden Mediums eingreifen und die Anwendung der Mikrodosiervorrichtung nach Ablauf einer Haltbarkeitsfrist für das Medium verhindern. Die mechanische und/oder elektronische Dosierverriegelung kann dabei insbesondere auf die manuell zu betätigende Dosierpumpe und/oder auf die Vibrationseinheit einwirken.

In weiterer Ausgestaltung der Erfindung ist eine Zähl- und/oder Anzeigevorrichtung für eine Dokumentation ausgetragener Mediumdosierungen vorgesehen. Eine Zähl- und/oder Anzeigevorrichtung kann mechanisch, elektrisch, elektronisch oder als Kombination davon ausgeführt sein. Mittels einer Zählvorrichtung kann eine Anzahl ausgeführter Mediumdosierungen aufgenommen und gespeichert werden. Für Dokumentationszwecke kann die gespeicherte Anzahl von Mediumdosierungen entweder über eine Auswertung des Speichers der Zählvorrichtung ausgelesen werden oder unmittelbar über eine Anzeigevorrichtung für den Benutzer zugänglich gemacht werden. Die Zählvorrichtung kann dabei als aufsteigender Zähler mit einem bei jeder Mediumdosierung zunehmenden Zählwert ausgeführt sein. Sie kann jedoch auch als absteigender Zähler mit einem bei jeder Mediumdosierung abnehmendem Zählwert ausgeführt sein. Insbesondere bei einem absteigenden Zähler kann somit eine Anzahl in der Mikrodosiervorrichtung gespeicherter Restmediumdosierungen auf einfache Weise dargestellt werden.

In weiterer Ausgestaltung der Erfindung sind Synchronisationsmittel für eine zeitlich abgestimmte Vibration in Abhängigkeit einer manuellen Betätigung der Dosierpumpe vorgesehen. Bei einer Förderung des Mediums aus dem Mediumspeicher in den Dosierraum mittels einer manuell zu betätigenden Dosierpumpe ist es vorteilhaft, wenn unmittelbar nach Betätigung der Dosierpumpe und dem damit verknüpften Mediumtransport in den Dosierraum gleich anschließend eine Mediumdosierung aus dem Dosierraum durch Betätigung der Vibrationseinheit stattfindet. Um diese aufeinanderfolgenden Vorgänge in vorteilhafter Weise miteinander zu kombinieren, sind Synchronisationsmittel vorgesehen, die insbesondere eine Betätigung der Dosierpumpe detektieren. Aus einer Detektion der Betätigung wird ein Steuersignal erzeugt, das an eine Steuereinrichtung der Vibrationseinheit weitergegeben wird. In der Steuereinrichtung wird durch das Steuersignal eine Vibrationsanforderung ausgelöst, die nach einer definierten Zeitspanne zu einer Ansteuerung der Vibrationseinheit und damit zur Mediumdosierung aus dem Dosierraum führt. Als Detektor für die manuelle Betätigung der Dosierpumpe können insbesondere Kontaktschalter, Durchflussmesser, Magnetschalter oder Druckmesser vorgesehen sein. Eine Steuereinrichtung für die Vibrationsmittel ist insbesondere als elektrischer oder elektronischer Schaltkreis ausgelegt. Mit Hilfe der Synchronisationsmittel wird sichergestellt, dass jeder manuellen Betätigung der Dosierpumpe auch eine synchronisierte Mediumdosierung aus dem Dosierraum folgt.

Vorteilhaft sind Gehäusebauteile des Mediumspeichers und des Energiespeichers aus Materialien einer einheitlichen Materialgruppe aufgebaut. Dies ist insbesondere vorteilhaft, wenn der Energiespeicher und der Mediumspeicher in der diskreten Verbrauchseinheit zusammengefasst sind. Die diskrete Verbrauchseinheit stellt vorzugsweise eine separate Gehäuseeinheit dar und wird einstückig entsorgt. Der Mediumspeicher ist zum Zeitpunkt der Entsorgung in der Regel zumindest nahezu vollständig entleert. Damit besteht für eine problemlose und ordnungsgemäße Entsorgung der Verbrauchseinheit die Hauptanforderung, den mit dem Mediumspeicher gekoppelten Energiespeicher umweltverträglich entsorgen. Da der Energiespeicher ohnehin Gehäusebauteile zur Ummantelung benötigt, ist es besonders vorteilhaft, wenn die für diese Ummantelung ohnehin verwendeten Materialien auch für die Gestaltung des Mediumspeichers und der den Mediumspeicher und den Energiespeicher umfassenden Gehäuseeinheit der Verbrauchseinheit verwendet werden. Selbst wenn für die unterschiedlichen Bestandteile des Mediumspeichers und des Energiespeichers nicht an allen Stellen identische Materialien verwendet werden können, ist es zumindest von Vorteil, wenn alle verwendeten Materialien einer einheitlichen Materialgruppe angehören. Dabei kommen insbesondere metallische Werkstoffe oder Kunststoffe in Frage.

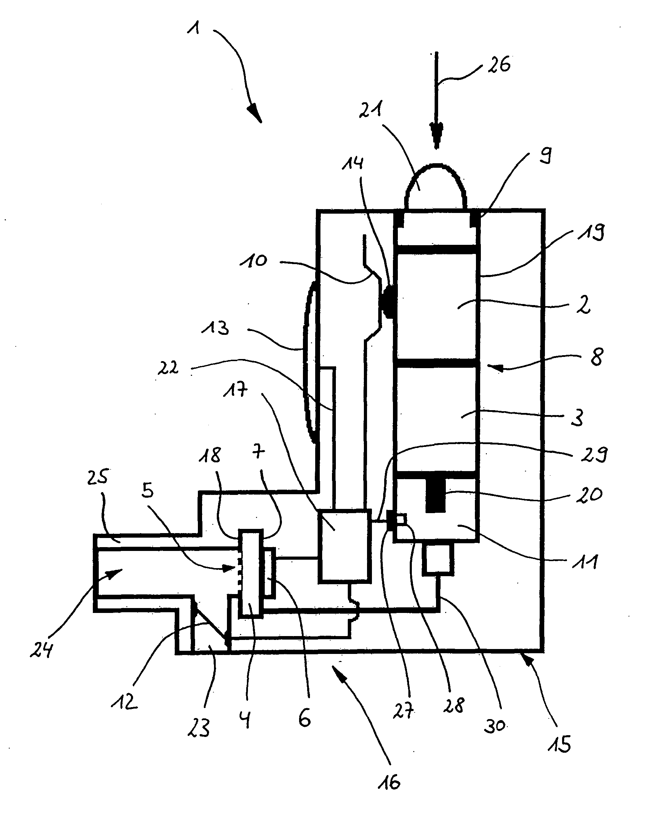

Weitere Vorteile und Merkmale ergeben sich aus den Ansprüchen sowie aus der nachfolgenden Beschreibung eines bevorzugten Ausführungsbeispiels der Erfindung, das anhand der einzigen Figur dargestellt ist.

Die einzige Figur zeigt in ebener Schnittdarstellung eine schematische Ansicht einer Mikrodosiervorrichtung .

Eine Mikrodosiervorrichtung 1 weist in einem Grundgehäuse 15 eine fest integrierte Zerstäubungseinrichtung 16 mit einer Ansteuervorrichtung 17 auf. Die Zerstäubungseinrichtung 16 ist aus einem als Ultraschallkammer 4 ausgeführten Dosierraum und einer als Ultraschallschwinger 6 ausgeführten Vibrationseinheit aufgebaut. Die Ultraschallkammer 4 weist dabei eine als Substrat 7 ausgeführte Begrenzungsfläche sowie eine Membran 18 auf, die als Membranporen 5 ausgeführte Austragöffnungen aufweist. Der Ultraschallschwinger 6 der Zerstäubungseinrichtung 16 wird von der Ansteuervorrichtung 17 mit elektrischer Energie versorgt. Die elektrische Energie wird in Abhängigkeit von Steuersignalen, die an der Ansteuervorrichtung 17 eintreffen, an den Ultraschallschwinger 6 weitergeleitet. Im Grundgehäuse 15 ist ein Aufnahmeschacht 19 vorgesehen, der zur Aufnahme einer eine Gehäuseeinheit in Form einer Kartusche 8 aufweisenden, diskreten Verbrauchseinheit vorgesehen ist. Die Kartusche 8 weist einen als Batterie 2 ausgeführten Energiespeicher sowie einen als Kunststofftank 3 ausgeführten Mediumspeicher auf. Die Kartusche 8 ist über eine elektromechanische Schnittstelle mittels Kontaktflächen 14 und als Kontaktzungen 10 ausgeführten Kontaktiermitteln mit der Ansteuervorrichtung 17 sowie über einen Anschlussstutzen 20 mit einer als Kolbenpumpe 11 ausgeführten Dosierpumpe verbunden. Die Kartusche 8 ist über als Rastnasen 9 ausgeführte Verriegelungsmittel lösbar mit dem Grundgehäuse 15 gekoppelt.

Der Aufnahmeschacht 19 wird durch einen Betätigungsverschluss 21 verschlossen, wobei der Betätigungsverschluss 21 die Ausübung einer Betätigungsbewegung auf die Kartusche 8 ermöglicht und somit die Auslösung einer Mediumdosierung zulässt. An dem Grundgehäuse 15 ist weiterhin eine als Zählwerk 13 ausgeführte Zähl- und Anzeigevorrichtung angebracht, die über eine Steuerleitung 22 mit der Ansteuervorrichtung 17 verbunden ist. An der Kolbenpumpe 11 ist ein Magnet 28 für die Ansteuerung eines Magnetschalters 27 vorgesehen, wobei der Magnetschalter über eine Signalleitung 29 mit der Ansteuervorrichtung 17 verbunden ist. An dem Grundgehäuse 15 ist ein Zuluftkanal 23 vorgesehen, der für die Zuführung von Umgebungsluft in einen Mischbereich 24 eines Mundstücks 25 vorgesehen ist. In dem Zuluftkanal 23 ist eine als Rückschlagklappe 12 ausgeführte Dosiertriggerung vorgesehen, die mit einer nicht dargestellten Feder in einer Schließstellung verharrt und lediglich bei einem Inhalationsvorgang durch einen Benutzer entgegen der Federvorspannung ausgelenkt wird.

Um eine Mediumdosierung aus der Mikrodosiervorrichtung 1 vorzunehmen, wird von einem nicht dargestellten Benutzer eine in Bedienrichtung 26 auf den Betätigungsverschluss 21 aufgebrachte Bedienkraft ausgeübt. Dazu wird insbesondere der Zeigefinger des Bedieners auf den Betätigungsverschluss 21 aufgelegt. Eine Abstützung der Mikrodosiervorrichtung 1 erfolgt an einer Unterseite des Grundgehäuses 15 durch den Daumen des Bedieners. Durch die Aufbringung der Betätigungskraft wird der Betätigungsverschluss 21 verschoben und erlaubt somit die Übertragung der Betätigungskraft auf die Kartusche 8. Die Kartusche 8, die die Batterie 2 und den Kunststofftank 3 umfasst, wird durch die Betätigungskraft ebenfalls in Bedienrichtung 26 verschoben und führt zur Erzeugung einer Pumpbewegung in der Kolbenpumpe 11. Nahezu zeitgleich mit der Ausübung der Betätigungskraft beginnt der Benutzer mit einem Inhalationsvorgang, zu welchem Zweck er das Mundstück 25 mit den Lippen fest umschließt und Luft durch den Zuluftkanal 23 ansaugt. Durch dieses Ansaugen wird die im Zuluftkanal 23 vorgesehene Rückschlagklappe 12 aus einer Ruheposition ausgelenkt und gibt somit ein elektrisches Triggersignal an die Ansteuervorrichtung 17 ab.

Bei zumindest nahezu gleichzeitigem Auftreten des Triggersignals und einem von der Kolbenpumpe ausgelösten Synchronisationssignal, das von dem Magnetschalter 27 und dem an der Kolbenpumpe befestigten Magnet 28 erzeugt wird, findet eine Aktivierung des Ultraschallschwingers 6 statt. Da gleichzeitig durch die Betätigung der Kolbenpumpe 11 die Ultraschallkammer 4 über eine Mediumleitung 30 mit Medium aus dem Kunststofftank 3 befüllt wird, kommt es durch die Schwingungen des Ultraschallschwingers 6 zu einer Druckwelle im Medium. Die Druckwelle führt zur Überwindung eines Strömungswiderstandes der Membranporen 5 der Membran 18, wodurch das Medium in Form von feinen Tröpfchen in den Mischbereich 24 ausgetragen wird. Da zeitgleich zu diesem Mediumaustrag ein durch den Inhalationsvorgang hervorgerufener Luftstrom im Mischbereich 24 vorherrscht, kommt es zu einer Durchmischung des ausgetragenen Mediums mit dem Luftstrom. Ausgetragenes Medium und Luftstrom können somit vom Benutzer durch das Mundstück 25 inhaliert werden und in den Rachenraum ,die Bronchien und/oder die Lunge des Benutzers weitergetragen werden. Gleichzeitig kommt es bei Betätigung des Betätigungsverschlusses 21 zu einer Ansteuerung des Zählwerks 13 über die Steuerleitung 22, wodurch ein angezeigter Zählwert verändert wird.

## Patentansprüche

1. Mikrodosiervorrichtung (1) für ein Medium mit einem Energiespeicher (2) für elektrische Energie, einem Mediumspeicher (3) und einem Dosierraum (4) zur zumindest zeitweisen Aufnahme einer Flüssigkeitsmenge, dem wenigstens eine Austragöffnung (5) und eine Vibrationseinheit (6) zugeordnet ist, die wenigstens mit einer Begrenzungsfläche (7) des Dosierraums (4) in Wirkverbindung steht, um diese für einen Austragvorgang in Schwingungen zu versetzen, **dadurch gekennzeichnet, dass** sich eine im Energiespeicher (2) gespeicherte Energiemenge und ein zum Austrag einer im Mediumspeicher (3) bevorrateten Mediummenge benötigter Energiebedarf zumindest nahezu entsprechen.

2. Mikrodosiervorrichtung nach Anspruch 1, **dadurch gekennzeichnet, dass** der Energiespeicher (2) und der Mediumspeicher (3) in einer diskreten Verbrauchseinheit (8) zusammengefasst sind.

3. Mikrodosiervorrichtung nach Anspruch 2, **dadurch gekennzeichnet, dass** die Verbrauchseinheit (8) lösbar mit einem Gehäuse der Mikrodosiervorrichtung verbunden ist.

4. Mikrodosiervorrichtung nach einem der vorhergehenden Ansprüche, **dadurch gekennzeichnet, dass** an dem Mediumspeicher (3) eine manuell zu betätigende Dosierpumpe (11) vorgesehen ist.

5. Mikrodosiervorrichtung nach einem der vorhergehenden Ansprüche, **dadurch gekennzeichnet, dass** eine mechanische und/oder elektrische Dosiertriggerung (12) zur Synchronisation der Dosierung mit einer Atembewegung eines Benutzers vorgesehen ist.

6. Mikrodosiervorrichtung nach einem der vorhergehenden Ansprüche, **dadurch gekennzeichnet, dass** eine mechanische und/oder elektronische Dosierverriegelung zur Sicherstellung einer korrekten Mediumdosierung vorgesehen ist.

7. Mikrodosiervorrichtung nach einem der vorhergehenden Ansprüche, **dadurch gekennzeichnet, dass** eine Zähl- und/oder Anzeigevorrichtung (13) für eine Dokumentation ausgetragener Mediumdosierungen vorgesehen ist.

8. Mikrodosiervorrichtung nach einem der Ansprüche 4 bis 8, **dadurch gekennzeichnet, dass** Synchronisationsmittel (27, 28) für eine zeitlich abgestimmte Vibration in Abhängigkeit einer manuellen Betätigung der Dosierpumpe (11) vorgesehen sind.
